# EUROPEAN PATENT APPLICATION

(11) **EP 1 854 430 A2**
(43) Date of publication of application: **14.11.2007**
(21) Application number: 07108092.3
(22) Date of filing: 11.05.2007
(51) Int. Cl.: A61F 2/30

(54) **Prosthesis having a rough articulation surface**

(30) Priority: 12.05.2006 GB 0609402
(71) Applicant: Finsbury (Development) Limited, Leatherhead, Surrey KT22 0BA (GB)
(72) Inventor: Dickinson, Alexander Stephen, East Molesey, Surrey KT8 9DE (GB); Taylor, Andrew Clive, Nr Chichester, Sussex PO19 3QQ (GB)
(74) Representative: Smaggasgale, Gillian Helen

(57) **Abstract**

A prothesis (1) having an articulating surface, wherein at least a portion (2) of said articulating surface is provided with a plurality of continuous grooves (3).

## Description

The present invention relates to a prosthesis. More particularly, it relates to a femoral head component for a hip prosthesis.

The efficient functioning of the hip joints is extremely important to the well being and mobility of the human body. Each hip joint is comprised by the upper portion of the femur which terminates in an offset bony neck surmounted by a ball-headed portion, the femoral head, which rotates within a socket, known as the acetabulum, in the pelvis. Diseases such as rheumatoid- and osteo-arthritis can cause erosion of the cartilage lining of the acetabulum so that the ball of the femur and the hip bone rub together causing pain and further erosion. Bone erosion may cause the bones themselves to attempt to compensate for the erosion which may result in the bone being reshaped. This misshapen joint may cause pain and may eventually cease to function altogether.

Operations to replace the hip joint with an artificial implant are well-known and widely practiced. Generally, the hip prosthesis will be formed of two components, namely: an acetabular component which lines the acetabulum and a femoral head component, which may be known as a stem component which replaces the femoral head. During the surgical procedure for implanting the femoral head component, the bone femoral head will be removed and the femur hollowed using reamers and rasps to accept the prosthesis. The femoral component will then be inserted into the femur. In some cases, the femoral head component described above may be replaced with components for use in femoral head resurfacing or for use in thrust plate technology.

As the present invention is concerned with the external surface of the femoral head itself, for ease of reference it will be referred to herein as a "femoral head component" but it will be understood that this will cover all femoral heads whether resurfacing prosthesis or full components with stem or any other component which includes the head.

Hip prostheses do not have an infinite life. A major factor influencing the life expectancy of a prosthetic implant is wear of the articulating surface which will generally be metal-on-metal wear. The pressures acting on the implant contact surface can be of the order of 30 MPa/cm² during normal function. Over time wear debris is formed as the components of the hip replacement articulate against one another. These particles can cause osteolysis and adverse tissue reactions which will lead to loosening of the component and reduce implant life. Currently the average life expectancy of a hip prosthesis is between 10 and 15 years. Where the implant has been placed in an elderly patient this period may be sufficient. However in a young patient, it is likely that one or more revision operations will have to be performed during the lifetime of the patient. It is therefore desirable to reduce wear rate and thereby improve implant life expectancy.

In existing implants, studies have observed high initial wear rate followed by a plateau at a stable, lower wear rate after about 10⁶ cycles. It may therefore be desirable to provide an implant which is either at this stable low wear rate at the time of insertion or in a much shorter space of time after insertion than has been achieved heretofore. It will be understood that in the prior art arrangement during the period of high wear, particles are dislodged from the surface of the prosthesis which in turn may increase the wear. Wear particles are believed to be responsible for up to 80% of abrasive wear in metal-on-metal articulation. It is therefore desirable to minimise the possibility of such particles being formed and/or providing a means for minimising the impact of such particles on the articulating surface.

Although the present invention is described in connection with a femoral head prosthesis it will be acknowledged, theat the invention is equally applicable to any other prosthesis for an articulating surface.

Thus according to the present invention there is provided a prosthesis having an articulating surface, wherein at least a portion of said articulating surface is provided with a plurality of continuous grooves.

The continuous grooves may be of any suitable shape. Suitable shapes include circles, irregular shapes and polygons having at least three sides. Particularly suitable polygons are hexagons. By "continuous" we mean that the grooves have no beginning and no end.

It will be understood that the various grooves on the surface may be of different shapes and sizes. That is to say not all grooves on the surface of the articulating surface have to be the same shape or size. For example, there may be at least two different configurations of the grooves present.

Without wishing to be bound by any theory, it is believed that lubricant compression and cavitation-limited decompression effects occur either side of a groove thereby generating increased lubricant film pressure which can promote fluid film lubrication.

The grooves may be of any suitable size. In general they will have a width in the range of 20 to 200 µm. They may have a depth of from 2 to 40 µm. Any suitable spacing of grooves may be used to achieve the desired fluid film lubrication. The grooves may be spaced at from 20 to 800 µm.

In one arrangement, small grooves may be present to improve fluid film lubrication and larger grooves which in use will trap any debris to prevent the debris causing wear. Suitable examples of "small" grooves include those having a width in the range of from about 20 µm to about 100 µm and a depth of from about 2 µm to about 20 µm. Suitable examples of "larger" grooves include those having a width in the range of from about 105 µm to about 200 µm and a depth of from about 25 µm to about 40 µm.

As the groove is continuous, the debris once trapped may move around the groove but will not generally be removed. Thus the debris will not interfere with the articulating surface and thus will not cause further wear thereof.

In a preferred arrangement the larger sized grooves can be introduced to surfaces under boundary and mixed lubrication.

The grooves of the present invention may be formed by any suitable method. In one arrangement, they may be formed by etching. In an alternative arrangement, the groove may be formed by building-up material which will be within the continuous groove and between the grooves i.e. the articulating surface. The building-up of material may be achieved by any suitable means including laser melting.

The positioning of the grooves and their spacing and size may be selected to provide differing effects on different areas of the surface of the prosthesis. In one arrangement the grooves may be concentrated is some areas of the prosthesis and other areas may have fewer grooves or may be free of grooves. In this connection it is noted that large plane areas are generally desirable for load support. In one arrangement there will be at least some grooves across the entire contact region of the articulating surface of the prosthesis.

The grooves may be formed on the present highly polished prosthesis. However, in a preferred arrangement, the prosthesis is not polished and the grooves are provided on the honed prosthesis, i.e. the state it is in when the manufacturing process is complete.

The prosthesis of the present invention is preferably a femoral head prosthesis.

The present invention will now be described, by way of example by reference to the accompanying drawings in which:
- Figure 1A: is a perspective view of a replacement femoral head prosthesis having the grooves of the present invention;
- Figure 1B: is an enlarged portion of a portion of the surface of the prosthesis of Figure 1A;
- Figure 2A: is a perspective view of a replacement femoral head prosthesis having the grooves of the present invention;
- Figure 2B: is an enlarged portion of a portion of the surface of the prosthesis of Figure 2A; and
- Figure 3: is a perspective view from above showing an alternative pattern of grooves.

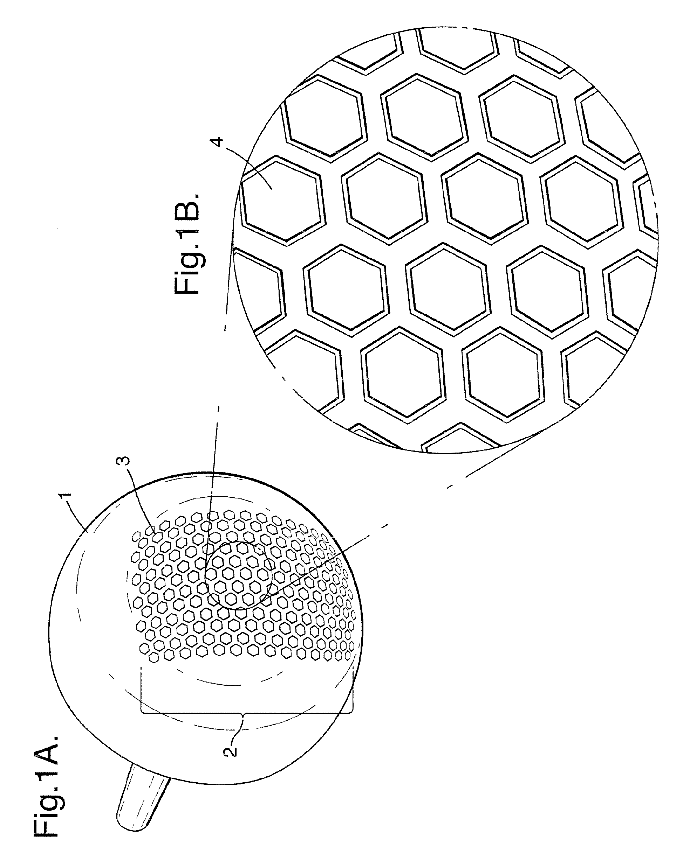

As illustrated in Figure 1A, a portion of the surface of the femoral head resurfacing prosthesis 1 is provided with a region 2 of grooves 3. As illustrated in Figure 1B, these grooves are continuous grooves in the shape of hexagons. It will be noted that a substantial proportion of planar surface 4 is retained.

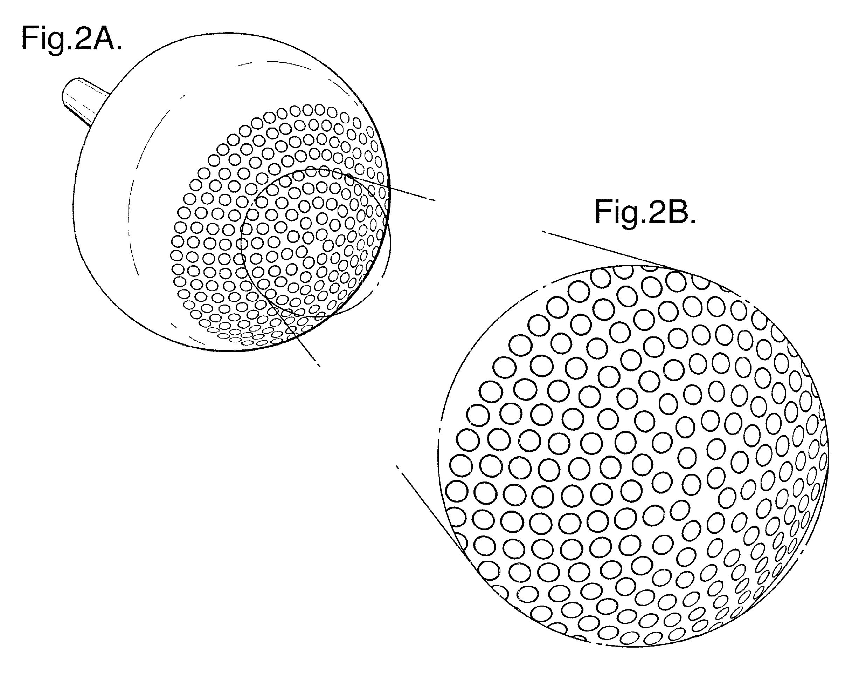

Alternative configurations of grooves may be used. Figures 2A and 2B illustrate the use of circular grooves.

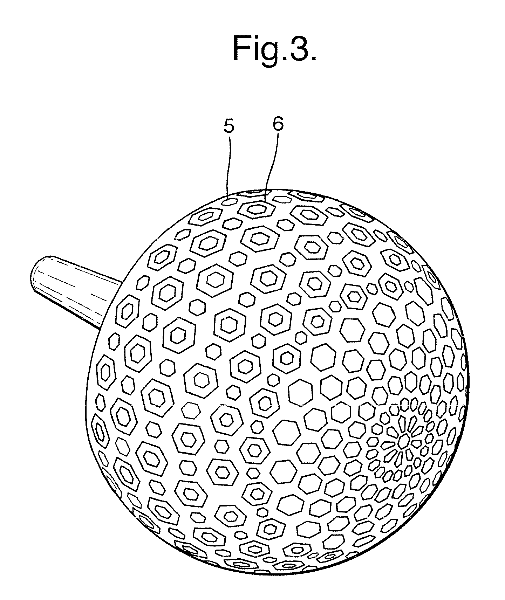

Figure 3 illustrates an alternative arrangement. Here a portion of the surface is covered with small hexagons 5 which are formed of smaller grooves and aid fluid lubrication. Larger grooves 6 which are generally deeper and broader are also provided to trap any debris.

Tests carried out using the prostheses of the present invention confirm that the wear profile is improved and the plateau of stable wear rate is achieved sooner than has been achievable heretofore.

## Claims

1. A prothesis having an articulating surface, wherein at least a portion of said articulating surface is provided with a plurality of continuous grooves.

2. A prothesis according to Claim 1, wherein the continuous grooves are circles, irregular shapes or polygons.

3. A prothesis according to Claim 2 wherein the polygons are hexagons.

4. A prothesis according to anyone of Claims 1 to 3 wherein all of the continuous grooves on the surface are of the same configuration.

5. A prothesis according to anyone of Claims 1 to 3 wherein there are at least two different configurations of continuous grooves of the articulating surface.

6. A prothesis according to anyone of Claims 1 to 5 wherein the plurality of continuous grooves are of substantially the same size.

7. A prothesis according to anyone of Claims 1 to 5 wherein there are at least two different sizes of continuous grooves.
